# EUROPEAN PATENT APPLICATION

(11) **EP 1 535 641 A1**
(43) Date of publication of application: **01.06.2005**
(21) Application number: 04027839.2
(22) Date of filing: 24.11.2004
(51) Int. Cl.: A61M 5/36, A61M 5/14

(54) **Drip chamber with air venting means**

(30) Priority: 28.11.2003 IT MO20030324
(71) Applicant: ARIES S.r.l., 41037 Mirandola (Modena) (IT)
(72) Inventor: Mantovani, Mauro, 41037 Mirandola (MO) (IT)
(74) Representative: Luppi, Luigi

(57) **Abstract**

Device for monitoring the flow rate of medical substances to a patient in an apparatus for administering said substances, comprising chamber means, drip conduit means arranged to deliver said substances to said chamber means according to preset and constant quantities, further conduit means arranged for alternatively placing said chamber means in communication with an ambient atmosphere or isolating it therefrom.

## Description

The invention relates to a monitoring device of the flow which is usable in apparatuses for the administration of medical substances, in particular apparatuses for the infusion of drugs.

Prior-art infusion apparatuses (infusion tubing with drip adjusters) comprise a tube comprising a supply end, equipped with a perforating element (spike), and a delivery end opposite the first end and provided with a needle to be inserted into the circulatory system of a patient. The medical substances to be administered may be liquid or lyophilized, in which case they are reconstituted with a suitable solvent at the moment of use and are normally available in containers (bags or bottles) the outlet conduits of which are closed by means of rubber diaphragms. By perforating the rubber diaphragm of the container of the medical substance with the perforating element, the medical substance penetrates into the tube and, through the needle inserted into the body of the patient, it is delivered inside the latter.

Prior-art apparatuses furthermore comprise a drip chamber, in which the tube terminates via a calibrated drip conduit. On the basis of the time that elapses between the fall of two successive drops into the drip chamber, it is possible to obtain a rough calculation of the flow rate of the medical substances into the infusion apparatus. Flow rate can be regulated by means of an adjuster placed between the drip chamber and the administration end of the apparatus, for example by means of a prior-art roller clamp that can be manually adjusted so as to modify the drug's flow area along the tube. In the length of tube comprised between the drip chamber and the administration end an injection point is provided that is equipped with a valve or with a perforable membrane through which it is possible to inject any supporting drugs.

During infusion of medical substances, for example antiblastic drugs, the apparatus tube may get clogged upstream of the drip chamber, with consequent emptying of the latter and of the length of tube comprised between the drip chamber itself and the administration end. As a result, the medical worker has to intervene to detach the administration end from the patient and compress the walls of the drip chamber to suck liquid from the medical substance container to the drip chamber, so as to refill the latter.

As the drip chamber has no transit paths for ambient air, the liquid must be sucked from the drip chamber by a syringe inserted into the withdrawal point in order to be able to completely fill the main length of tube comprised between the drip chamber and the administration end. In order to completely remove any air bubbles from the tube, the operator has to carry out priming, i.e. he has to continue to suck liquid from the drip chamber until he makes some drops emerge from the administration end. At this point, the operator can reconnect the infusion apparatus to the patient and resume administration.

The procedure disclosed above, used in prior-art infusion apparatuses to restore the accidentally interrupted drug flow, is extremely disadvantageous for various reasons.

First of all, it is known that a considerable number of antiblastic drugs have high collateral toxicity that may for example cause irritation to the skin and/or mucous membranes in the event of accidental contact and in certain cases even blisters or actual ulcers. This implies a considerable risk of chemical contamination to which both the patient receiving chemotherapeutic treatment and the medical personnel physically involved in the administration of the latter are exposed.

Such a risk becomes real when the medical worker has to complete the priming of the tube disclosed above, thereby releasing albeit minimal quantities of the drug into the environment.

Furthermore, antiblastic drugs are extremely costly and the corresponding doses are intended to achieve certain therapeutic effects. Thus releasing into the environment even minimal volumes of these drugs is economically disadvantageous and harmful for the therapeutic efficacy of the administration.

Lastly, the task that the operator has to perform to disconnect and reconnect the patient from and to the infusion apparatus substantially prolongs the duration of administration and makes the therapeutic treatment, which is in itself unpleasant for the patient subjected to it, even more trying.

An object of the present invention is to improve the apparatuses for infusion of medical substances.

A further object is to create infusion apparatuses of medical substances in which, in the event of an interruption to infusion due for example to an accidental obstruction, it is possible to restore the flow of liquid, thus avoiding the release of medical substances into the environment.

The invention provides a device for monitoring the flow rate of medical substances to a patient in an apparatus for administering said substances, comprising chamber means, drip conduit means arranged to deliver said substances to said chamber means according to preset and constant quantities, further conduit means arranged to alternatively make said chamber means communicate with an ambient atmosphere or isolate it therefrom.

Owing to the invention it is possible to restore the flow of a medical substance into an infusion apparatus, thus preventing undesired leakage of the medical substance outside the apparatus: in fact, as will be disclosed in greater detail below, having provided for the drip chamber being able to communicate with the outside enables a desired quantity of drug in the length of tube comprised between the drip chamber and the administration end to be restored without having to perform the priming operation disclosed above.

The invention will be better understood and implemented with reference to the enclosed drawings that show some exemplifying and non-limitative embodiments thereof, in which:
Figure 1 is a partially sectioned longitudinal view of a device according to the invention;
Figure 2 is a front view of a device according to the invention inserted into an apparatus for the infusion of medical substances;
Figure 3 is a partially sectioned longitudinal view of a further embodiment of the device illustrated in Figure 1.

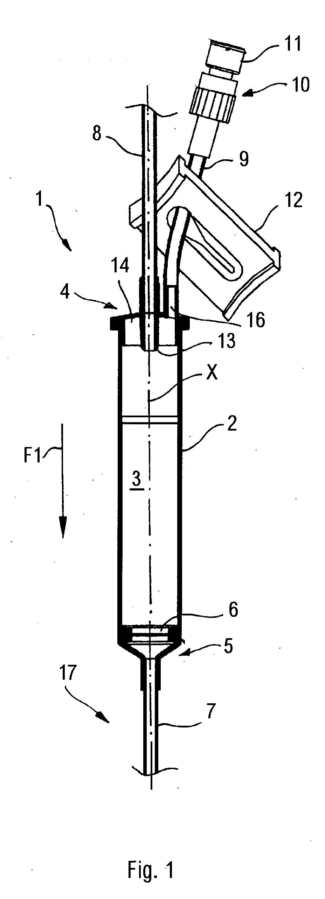

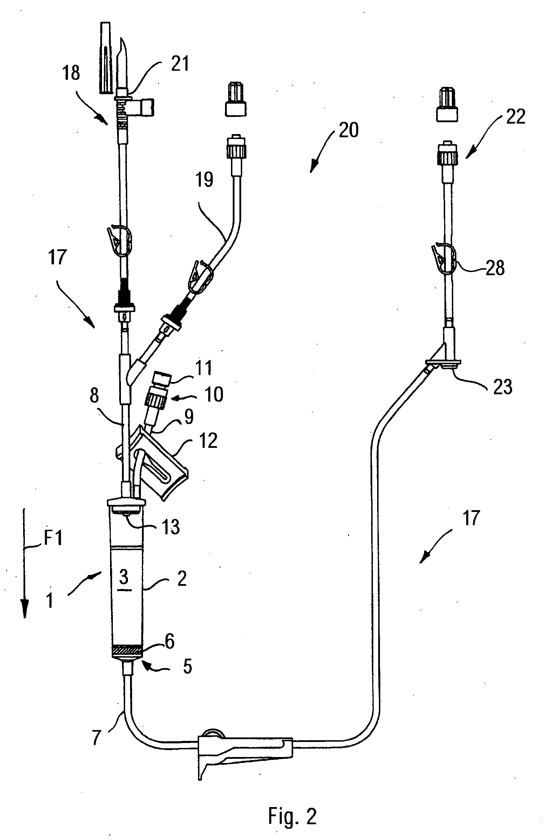

In Figures 1 and 2, a drip chamber 1 arranged to be traversed by a liquid medical substance that is not shown in a direction indicated by the arrow F1 comprises a wall 2 defining a substantially cylindrical internal cavity 3. The chamber 1 furthermore comprises a supply end 4 and an administration end 5 that is cone-shaped and opposite the supply end 4. Near the administration end 5 the internal cavity 3 receives a first filtering element 6, the peripheral edge of which is internally juxtaposed to the wall 2, which filtering element 6 is arranged to filter the medical substance before the latter comes out of the internal cavity 3 through an outlet length 7 of a main supply line 17 of the medical substance forming part of an infusion apparatus 20. The supply end 4 is closed by a lid element 14, which is approximately cone-shaped and receives an inlet length 8 of the main supply line 17 and a transit conduit 9 for the gases, for example ambient air. The inlet length 8 is inserted into a drip conduit 13, which is cylindrical and substantially aligned on a longitudinal axis X of the chamber 2, whereas the transit conduit 9 is inserted into a cylindrical joint conduit 16 arranged in a peripheral position in relation to the drip conduit 13. The section of the conduit 13 is realized in such a way as to the liquid medical substance coming from the inlet conduit 8 produces, by flowing into the internal chamber 3, drops having a preset and constant volume. In particular, the drip conduit 13 can be made in such a way as to produce so-called 'standard' drops or 'microdrops'. The difference in volume between a standard drop and a microdrop is such that for example 1 millilitre of liquid corresponds to 20 standard drops or to 60 microdrops.

The transit conduit 9 is provided at an end opposite the supply end 4 with an end joint 10, for example of the "Luer Lock" type, with which a second filtering element 11 is associated. The latter is prior-art, water-repellent and has porosity comprised in a range of values from 0.02 µm to 10 µm. The transit conduit 9 is furthermore provided with a section reducer 12, for example a prior-art safety clamp that is manually operable by an operator to narrow the section of the conduit 9 until the latter is closed and to thus separate the internal cavity 3 from the ambient air.

When the infusion apparatus 20 equipped with the drip chamber 1 disclosed above is used, by means of a spike 21 associated with a supply end 18 of the main supply line 17 the rubber diaphragm is perforated that shuts the inlet of a container (not shown) of a medical substance to be administered, thus enabling the latter to enter the supply line 17 and to reach the drip chamber 1. From the drip chamber 1, which displays the flow rate of the medical substance, the latter is conveyed to an administration end 22 provided with a needle that is not shown through which the medical substance is injected into a patient's circulatory system. By means of a further supply line 19, connected to the supply line 17 upstream of the drip chamber 1 and also equipped with a perforating element (not shown) at a free end, it is possible to take a further medical substance to be delivered to the main line 17 from a further container that is not shown. The flow rate of the medical substance conveyed through the main supply line 17 is visually monitorable by means of the drip chamber 1 and can be adjusted by acting on the cross-section of the main supply line 17 by a flow adjuster 27, for example a prior-art roller clamp. If the flow into the main supply line 17 upstream of the drip chamber 1 is interrupted after an obstruction or because the substance to be administered has finished, the drip chamber 1 empties together with a length of the main supply line 17 comprised between the administration end 22 and the chamber 1. The assigned medical worker can then intervene by closing by means of a further prior-art section reducer 28, for example a clamp, a length of main supply line 17 comprised between the administration end 22 and an injection point 23 provided with a valve or perforable membrane or with a tap (not shown). Then, he open the transit conduit 9 by manually acting on the section reducer 12 with a consequent flow of ambient air, filtered by the second filtering element 11, into the internal cavity 3. This enables the medical substance, after removal of the obstruction, if current, or after connecting the apparatus to a new container of medical substance still to be administered, together with ambient air, to fill the drip chamber 1. To resume administration, it is sufficient to remove through the transit conduit 9 any air that may have entered the outlet length 7. This is obtained by the operator by injecting physiological solution into the main supply line 17 via the injection point 23. The physiological solution rises against the current in the outlet length 7 to the drip chamber 1, pressing into the latter the air bubbles current therein. The gas bubbling in the liquid that fills the drip chamber 1 comes out from the internal cavity 3 and passes into the ambient air through the transit conduit 9. The ceasing of bubbling in the drip chamber 1 indicates to the operator that all the air has been expelled from the apparatus, which enables the transit conduit 9 to be closed by means of the section reducer 12, the main supply line 17 downstream of the withdrawal point 23 to be reopened by acting on the further section reducer 28 and administration of the medical substance to be thus resumed.

By following the procedure disclosed above it is thus possible to restore the flow of medical substance into the infusion apparatus without resorting to priming, which is inappropriate for reasons of safety, cost and reduced efficacy of the therapeutic treatment.

Furthermore, it is not necessary to disconnect and subsequently reconnect the patient to the infusion apparatus, thus sparing the latter from trying prolongation of the duration of therapy.

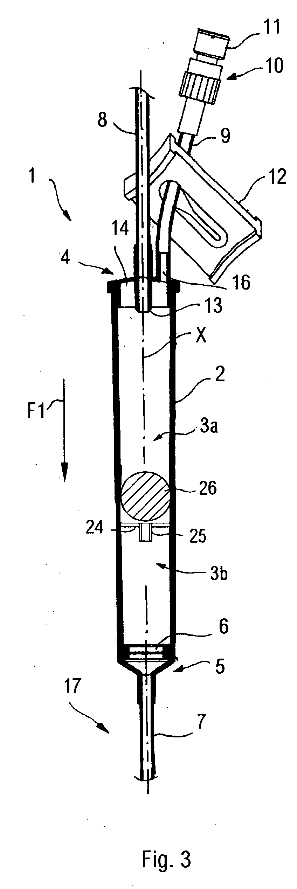

Figure 3 shows a further embodiment of the drip chamber 1 in which the internal cavity 3 is divided by a perforated diaphragm 24 into a proximal portion 3a, comprising the supply end 4, and a distal portion 3b, comprising the administration end 5. The perforated diaphragm 24 is traversed by a cylindrical connecting conduit 25 that is essentially aligned on the longitudinal axis X of the chamber 1, which extends from the perforated diaphragm 25 in the direction of the first filtering element 6 and makes the proximal portion 3a communicate with the proximal portion 3b. In the proximal portion 3a a closing element 26 is received that is sphere-shaped and has a diameter that is such that it is movable in the direction of the longitudinal axis X inside the proximal portion 3a.

By using this further embodiment of the drip chamber 1 in the infusion apparatus 20 the administered medical substance fills the proximal portion 3a by making the closing element 26 float and keeping the latter at a distance from the perforated diaphragm 24. When the administration has been completed, the proximal portion 3a empties, the closing element 26 is no longer supported by a liquid mass and falls back onto the perforated diaphragm 24, thus obstructing the conduit 25. In this way, the air that is in the container of the medical substance and that has penetrated into the chamber 1 through the main supply line 17 cannot continue further along the latter to reach the patient's circulatory system. This undesired eventuality may occur if the medical worker does not intervene promptly to close by means of the roller clamp 27 the main supply line 17 downstream of the drip chamber 1.

The drip chamber 1 may be made of a material that is chemically resistant to the medical substances to be administered. In this way, undesired chemical components are not released inside the chamber even when particularly corrosive medical substances such as certain types of antiblastic drugs traverse the chamber.

In particular, the chemically resistant material can be selected from a group comprising: polyvinyl chloride free of phthalates ("PVC DEHP free"), polyurethane (PUR), polyethylene (PE), polypropylene (PP), ethyl vinyl acetate (EVA).

In a further embodiment, the drip chamber may be made of a photoprotective material comprising pigments that are able to block luminous radiation having a wavelength comprised between 250 and 500 nm. This enables the chamber to be used also for administering photosensitive drugs the chemical structure of which is altered by exposure to ambient luminous radiation, with consequent reduction of the respective pharmacological activity. The photoprotective material may for example be amber in colour, and this allows medical personnel to easily and immediately distinguish a drip chamber according to the invention that is usable for administering photosensitive drugs from similar devices that are not made of photoprotective material.

The drip chamber 1 disclosed above may be used in infusion apparatuses for administering many different antiblastic drugs, such as for example: Bleomycin, Carboplatinum, Carmustin, Ciclophosphamide, Cisplatinum, Citarabine, Cladribine, Dacarbazine, liposomial citrate Daunorubicin, Daunorubicin hydrochloride, Docetaxel, Doxorubicin hydrochloride, Epirubicin, Etoposide, Fludarabine, Fluorouracil (5 FU), Gemcitabine hydrochloride, Ifosfamide, Irinotecan hydrochloride, Mechlorethamine hydrochloride, Melphalan hydrochloride, Methotrexate sodium, Mitomycin, Mitoxantrone hydrochloride, Paclitaxel, Thiotepa, Vinblastine sulphate, Vincristine sulphate, Vindesine sulphate, Vinorelbine tartrate.

## Claims

1. Device (1) for monitoring the flow rate of medical substances to a patient in an apparatus (20) for administering said substances, comprising chamber means (2), drip conduit means (13) arranged to deliver said substances to said chamber means (2) according to preset and constant quantities, further conduit means (9) arranged to alternatively make said chamber means (2) communicate with or be isolated from an ambient atmosphere.

2. Device according to claim 1, wherein said chamber means 2 comprises an internal cavity (3) comprised between a supply end (4) and an administration end (5) opposite each other.

3. Device according to claim 2, wherein said administration end (5) is provided with first filtering means (6).

4. Device according to claim 3, wherein said first filtering means (6) is arranged in said internal cavity (6).

5. Device according to any one of claims 2 to 4, wherein said drip conduit means (13) is associated with said supply end (4).

6. Device according to claim 5, wherein said drip conduit means (13) is substantially aligned on a longitudinal axis (X) of said chamber means (2).

7. Device according to any one of claims 2 to 6, wherein said further conduit means (9) is associated with said supply end (4).

8. Device according to claim 7, wherein said further conduit means (9) is associated with a peripheral portion of said supply end (4).

9. Device according to claim 7, or 8, wherein said further conduit means (9) is associated with joint means (16) obtained in said supply end (4).

10. Device according to any preceding claim, wherein an end of said further conduit means (9) opposite said drip chamber (2) is provided with second filtering means (11).

11. Device according to claim 10, wherein said second filtering means (11) comprises a filter (11).

12. Device according to claim 11, wherein the porosity of said filter (11) is approximately comprised in a range of values from 0.02 µm to 10 µm.

13. Device according to claim 8, wherein said second filtering means (11) comprises a sponge.

14. Device according to any preceding claim, wherein said further conduit means (9) comprises section reducing means (12).

15. Device according to claim 14, wherein said section reducing means (12) comprises a safety clamp (12).

16. Device according to any one of claims 2 to 15, furthermore comprising perforated diaphragm means (24) arranged inside said internal cavity (3) and such as to divide the latter into a proximal portion (3a) and a distal portion (3b) communicating with each other.

17. Device according to claim 16, wherein said perforated diaphragm means (24) is furthermore provided with connecting conduit means (25).

18. Device according to claim 17, wherein said connecting conduit means protrudes from said perforated diaphragm means (24) towards said administration end (5).

19. Device according to any one of claims 16 to 18, and furthermore comprising closing means (26) received in said proximal portion (3a).

20. Device according to claim 19, wherein said closing means (26) comprises a sphere-shaped closing element (26).

21. Device according to any preceding claim, wherein said chamber means (2) is made of a material that is chemically resistant to said medical substances.

22. Device according to claim 21, wherein said material is selected from a group comprising: polyvinyl chloride free of phthalates ('PVC DEHP free'), polyurethane (PUR), polyethylene (PE), polypropylene (PP), ethyl vinyl acetate (EVA).

23. Device according to any preceding claim, wherein said chamber means (2) is made of a photoprotective material.

24. Device according to claim 23, wherein said photoprotective material comprises pigments such as to block luminous radiation having a wave length comprised between 250 and 500 nm.

25. Device according to claim 23, or 24, wherein said photoprotective material is amber in colour.

26. Use of an apparatus for infusion of medical substances comprising a device according to any one of claims 2 to 25.
